# EUROPEAN PATENT APPLICATION

(11) **EP 1 876 449 A1**
(43) Date of publication of application: **09.01.2008**
(21) Application number: 06014144.7
(22) Date of filing: 07.07.2006
(51) Int. Cl.: G01N 33/68

(54) **Cell cycle-based blood test to diagnose Alzheimer's disease**

(71) Applicant: Universität Leipzig, 04109 Leipzig (DE)
(72) Inventor: Arendt, Thomas, 04229 Leipzig (DE); Stieler, Jens, 01309 Dresden (DE); Gartner, William J., Arizona 85253 Paradise Valley (US); Grimes, F. Randall, Arizona 85254 Scottsdale (US); Weber, Donald F., Arizona 85262 Scottsdale (US)
(74) Representative: Schüssler, Andrea

(57) **Abstract**

Described is a method of diagnosing Alzheimer's Disease (AD), an early stage or predisposition for said disease in a blood sample obtained from a patient comprising: (a) quantitatively determining (i) expression of blood cell surface marker CD69 prior to and after stimulation of the cells of the blood sample by a mitogen and (ii) the ratio of CD45RO/CD45RA; (b) comparing the expression values of said surface markers of the blood cells with referenc expression values of the same surface markers; and (c) relating the determined expression of CD69 and the ratio of CD45RO/CD45RA to an assessment of the risk of developing the disease, a diagnosis of the disease, or a measure of the progression of the disease. Also described are methods for distinguishing between AD patients and non-AD patients, preferably Parkinson's Disease (PD) patients.

## Description

The present invention relates to a method of diagnosing Alzheimer's Disease (AD), an early stage or predisposition for said disease in a patient blood sample comprising: quantitatively determining (i) expression of blood cell surface marker CD69 prior to and after stimulation of the cells of the blood sample by a mitogen and (ii) the ratio of CD45RO/CD45RA; (b) comparing the expression values of said surface markers of the blood cells with reference expression values of the same surface markers and (c) relating the determined expression of CD69 and the ratio of CD45RO/CD45RA to an assessment of the risk of developing the disease, a diagnosis of the disease, or a measure of the progression of the disease. The present invention also relates to methods for distinguishing between AD patients and non-AD patients, such as patients having Parkinson's Disease (PD), dementia with Lewy bodies, Fronto-Temporal Dementia and Vascular Dementia.

The early diagnosis of Alzheimer's disease (AD) is the basis for a successful medical intervention in AD extending or maximizing the patient's quality of life (1;2). However, clinical diagnosis does not always attain adequate accuracy levels especially in detecting early stages of AD or in differentiating it from other forms of dementia (3;4). Problems also exist with the time and costs necessary for differential diagnosis. Accordingly, only confirmation by postmortem neuropathology is considered a definitive diagnosis of AD. To increase sensitivity and specificity, clinical diagnosis should be based on cumulative information, including neuropsychological examination and the analysis of disease specific biological markers (5;6).

Besides the more frequently applied brain imaging techniques, the investigation of cerebrospinal fluid as well as peripheral body fluids and tissues plays an important role in the search for a clinically relevant marker (7). The search for a biological marker for Alzheimer's disease (AD) has been driven by dissatisfaction with the current clinical diagnosis. In general, criteria for clinical diagnosis of AD are based on exclusion of other forms of dementia as outlined by the NINCDS-ADRDA work group (22). Sensitivity and specificity for clinical diagnosis, mainly depending on the stage of the disease and the experience of the clinician, can attain high rates of 80% to 90% (21), although it is frequently noted that outside of qualified academic centres such as at the primary care level accuracy rates will be notable decreased (3;23;35).

This indicates the essential need for a biological marker for AD diagnosis that in particular should be a helpful tool in early detection and definite exclusion of other forms of dementia. The criteria for AD biomarkers are outlined in the report of a consensus group (8) proposing that an AD biomarker should have, both, diagnostic sensitivity and specificity of more than 80% while detecting a fundamental feature of the neuropathology. It should be non-invasive, reliable, reproducible, inexpensive and easy to perform.

The search for an AD-biomarker is limited by the fact that the cause of AD is still unknown, although research of recent years could shed light into specific aspects of the disease. Investigations for a valid biomarker, therefore, are mainly focused on the major pathological hallmarks such as brain atrophy related to a neurodegenerative process associated with typical albeit not entirely specific molecular abnormalities such as formation of the Aβ-peptide and hyperphosphorylated tau. More recently, cell cycle dysregulation has been identified as critically associated with neurodegeneration (for review, see (26)).

Brain atrophy such as for example in the hippocampus, a site of early degeneration, requires a certain extent to become detectable by imaging techniques which only occurs beyond the initial stages of the disease. Significant costs and restricted availability of imaging techniques based on alterations of blood flow, volumetric or metabolic alterations, furthermore, limit their application for early diagnosis. Moreover, sensitivity and specificity of these procedures are hardly in the range of above 80%. Similar limitations in sensitivity and specificity apply to current tests based on the detection of Aβ or tau protein in the cerebrospinal fluid. Sensitivity of both markers might reach values of around 80% while specificity is well below that showing considerable overlap with other dementia disorders or even controls. For the detection of phosphorylated species of the tau protein in CSF, reported specificity with values of up to 85% is somewhat higher while at the same time sensitivity is decreased. Diagnostic accuracy can potentially be improved combining more than one CSF biomarker. Still, in a multicentre study, combining the analysis of Aβ1-42 and tau protein, sensitivity only reached 85% while specificity to distinguish AD from non AD-types of dementia was even below 60% (27). Lumbar puncture, furthermore, is not a technique routinely applied at the primary care level and does not comply with the NIA working groups recommendations for non-invasive procedures as it imposes additional risk to the patient. In conclusion, there is neither an imaging technique nor a CSF biomarker available suitable to perform large scale diagnostic testing at the primary care level.

In contrast to CSF, blood plasma or serum would be obviously preferred as a source for a biological AD marker. While some of the reported plasma or serum markers show significant differences between AD patients and control groups, they all lack sensitivity and specificity. At present, no single biomarker or panel of biomarkers has been shown to be successful as an adjunct to or potential replacement for the clinical diagnosis of AD.

Thus, the technical problem underlying the present invention is to provide an improved method for the reliable diagnosis of AD even allowing detecting (very) early stages of the disease.

The solution to said technical problem is achieved by providing the embodiments characterized in the claims. The study resulting in the present invention was based on evidence for a critical involvement of cell cycle dysregulation in AD that potentially shows a systemic character and is also manifested in peripheral tissues and, in particular, in blood cells (13-20). It was tested whether a biomarker profile, generated by the simultaneous determination of several disease specific biomarkers on lymphocytes which basically reflect the status of cell cycle activation upon mitogenic stimulation, correlates to the clinical diagnosis of AD. Due to the associated expense and the limited availability of imaging techniques and lumbar puncture at both primary care level and geriatric offices, blood was used as source for AD biomarker. Venous puncture is a generally accepted procedure that is defined as a non-invasive procedure by the FDA. Therefore, a blood-based test would be favorable, provided that such a test complies with conditions for an "ideal" AD biomarker as outlined in the Consensus Report of the Working Group on: "Molecular and Biochemical Markers of Alzheimer's Disease" (8).

Primarily based on findings suggesting a critical role for cell cycle dysfunction in AD pathogenesis, a biomarker profile could be generated which shows a co-positivity of 91% and a co-negativity of 92% relative to the clinical diagnosis of AD. This high level of agreement with the clinical diagnosis demonstrates the test's suitability as an improved and reliable diagnostic tool for AD.

In the present, cross-sectional, controlled trial 32 subjects with clinically diagnosed probable AD, 26 demented subjects diagnosed with Parkinson's disease and 30 non-demented subjects were studied. The expression of 10 leukocyte antigens, either at their basal level or after mitogenic stimulation, was determined by flow cytometry. Based on the expression profile, scoring models were calculated that subsequently were tested with regard to a correlation to the clinical diagnosis of study subjects. From all subjects we obtained adequate measurements. 17 out of 52 evaluated or derived markers were found significantly different in AD subjects from non-AD subjects. Depending on the applied calculated scoring model, it could be differentiated between AD subjects and other dementia (OD) subjects (such as Parkinson's Disease (PD), dementia with Lewy bodies, Fronto-Temporal Dementia and Vascular Dementia) with co-positivity up to 91% and a co-negativity up to 92% relative to the clinical diagnosis of AD.

Thus, this test shows a very strong correlation to the clinical diagnosis of AD and holds promise as either an adjunct to, or potential replacement for, the differential diagnostic procedures presently used.

### Brief description of the drawings

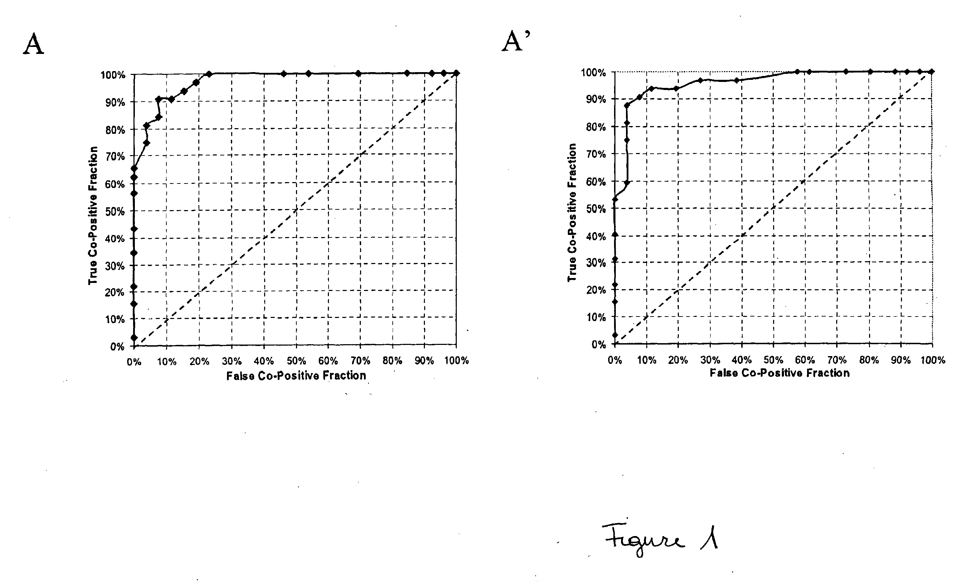

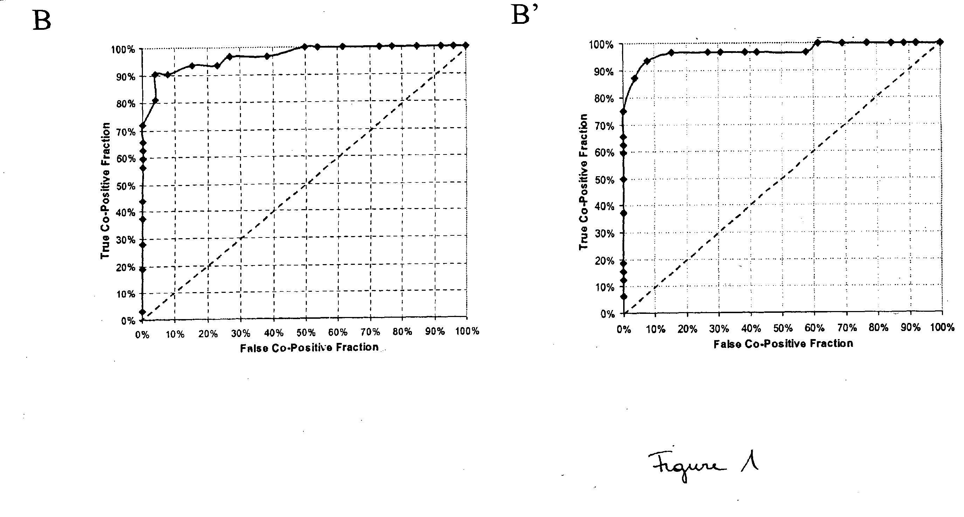

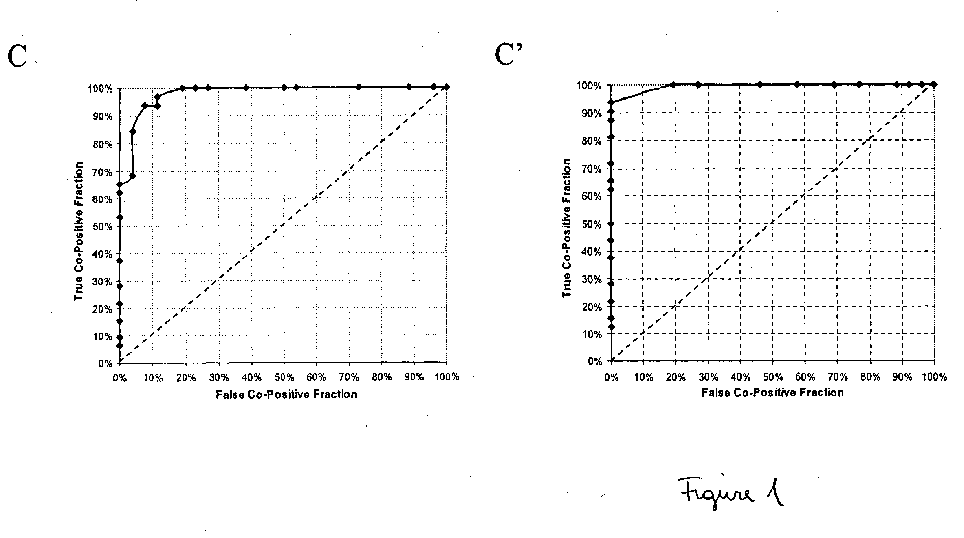

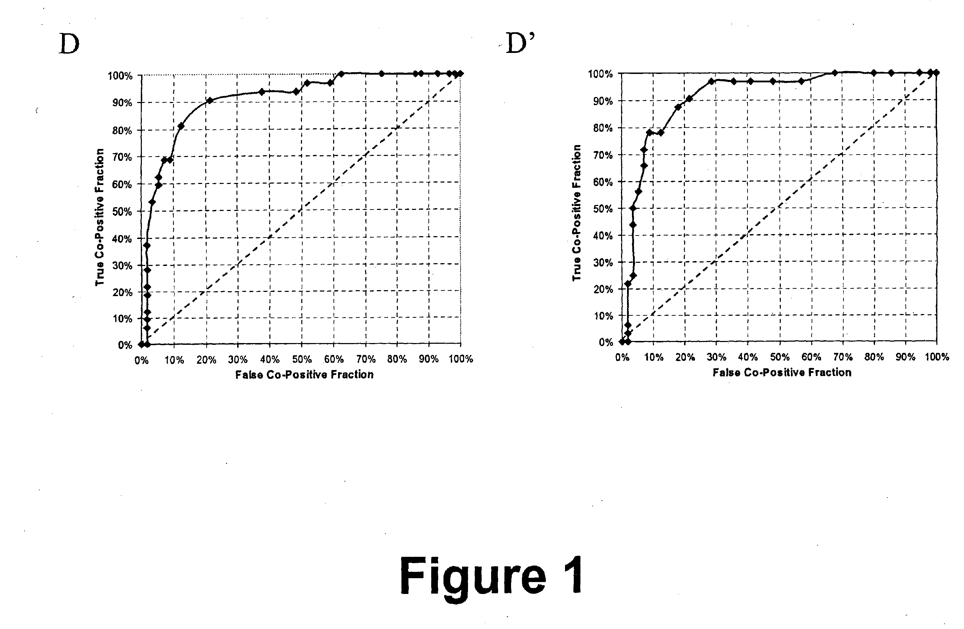

### Figure 1: Receiver operator characteristic curves

The curves were generated using raw (A) and normalized (A') total data set scoring model, raw (B) and normalized (B') PHA restricted data set scoring model and raw (C) and normalized (C') PWM restricted data set scoring model differentiating AD from PD as well as raw (D) and normalized (D') total data set scoring model differentiating AD from non-AD.

Accordingly, the present invention provides a method of diagnosing Alzheimer's Disease (AD), an early stage or predisposition for said disease in a blood sample obtained from a patient comprising:
(a) quantitatively determining (i) expression of blood cell surface marker CD69 prior to and after stimulation of the cells of the blood sample by a mitogen and (ii) the ratio of CD45RO/CD45RA expression;
(b) comparing the expression values of said surface markers of the blood cells with reference expression values of the same surface markers; and
relating the determined expression of CD69 and the ratio of CD45RO/CD45RA to an assessment of the risk of developing the disease, a diagnosis of the disease, or a measure of the progression of the disease.

Alternatively, the present invention provides a diagnostic method for distinguishing between AD and a different chronic progressive Non-Alzheimer's Dementia (such as Parkinson's Disease (PD), dementia with Lewy bodies, Fronto-Temporal Dementia and Vascular Dementia) comprising:
(a) quantitatively determining (i) expression of blood cell surface marker (a) CD69 prior to and after stimulation of the cells of the blood sample by a mitogen and (ii) the ratio of CD45RO/CD45RA;
(b) comparing the expression values of said surface markers of the blood cells with reference expression values of the same surface markers; and
relating the determined expression of CD69 and the ratio of CD45RO/CD45RA for distinguishing between AD and a different chronic progressive Non-Alzheimer's Dementia.

The person skilled in the art knows suitable methods for taking blood samples from a patient, also knows how such samples should be processed for further analyses and is familiar with mitogens that can be used for stimulating mitogenesis of blood cells.

In the present study, 40 surface markers having diagnostic relevance were determined based on the results of flow cytometry analyses. Each individual marker was assayed for significant changes of expression compared to the expression values found in the various groups of test persons (AD vs. PD or AD vs. Non-AD). Markers showing a significant change were combined in scoring models. The numerical values of these scoring models were used for diagnostic evaluation. In most of these models the cut-off points (comprising the evaluation of multiple markers) were given as about 0.5. This means that subjects having a score of > 0.5 are suffering from AD whereas subjects having a score of < 0.5 are not suffering from AD.

The term "reference expression value" as used herein, characterizes a value obtained from a single marker. With respect to the evaluation of surface markers responding to mitogenic stimulation (e.g., CD69) the "reference expression value" means an "internal" reference value, which was obtained from the same patient and which characterizes the normal status of the immune cells. Thus, this reference expression value means a value obtained from a sample that was not stimulated by a mitogen but that was incubated together with the stimulated sample under the same experimental conditions (non-stimulated sample). The extent of the stimulation (activation) (activity index; AI) is the ratio of the concentration of CD69/stimulated sample : CD69/non-stimulated sample. The term "reference expression value" with respect to the evaluation of surface markers which are not stimulated by a mitogen (e.g., CD28, CD45RO, CD45RA) means an reference expression value obtained from healthy patients, i.e., patients that are cognitively normal, do not show any signs of any dementia and show as regards CD69 expression a normal mitogenic response, have a "normal" CD28 expression or "normal" ratio of CD45RO/CD45RA. The term "normal" as used herein corresponds to a value which was determined by using the average values obtained from 30 individuals (see Tables 1 and 2 for cutoff-points).

| Table 1: Markers differentiating Alzheimer's disease from non-Alzheimer's disease (diagnostic screening). | | |
|---|---|---|
| **Marker** | **cutoff-points** | |
| | **control** | **AD** |
| Percentage of CD45RO+\|CD3+ | < 55% | > 55% |
| AI2 of CD4+ T-cells after PWM stimulation | > 3,0 | < 3,0 |
| AI1 of CD4+ T-cells after PHA stimulation | > 8,0 | < 8,0 |
| AI1 of CD8+ T-cells after PWM stimulation | > 2,0 | < 2,0 |
| AI2 of CD8+ T-cells after PHA stimulation | > 3,0 | < 3,0 |
| AI1 of CD14+ cells after PWM stimulation | > 4,0 | < 4,0 |
| AI1 of CD14+ cells after PHA stimulation | > 4,0 | < 4,0 |
| AI2 of CD14+ cells after PHA stimulation | > 1,5 | < 1,5 |
| AI2 of CD14+ cells after PWM stimulation | > 1,5 | < 1,5 |
| AI1 of CD19+ cells after PWM stimulation | > 3,5 | < 3,5 |
| AI2 of CD19+ cells after PWM stimulation | > 4,5 | < 4,5 |
| AI2 of CD 19+ cells after PHA stimulation | > 4,5 | < 4,5 |

| Table 2: Markers differentiating Alzheimer's disease from other dementias (differential diagnosis). | | |
|---|---|---|
| **Marker** | **cutoff-points** | |
| | **control** | **AD** |
| AI1 of CD8+ T-cells after PWM stimulation | > 2,0 | < 2,0 |
| AI1 of CD14+ cells after PWM stimulation | > 4,0 | < 4,0 |
| AI2 of CD 14+ cells after PHA stimulation | > 1,5 | < 1,5 |
| AI2 of CD14+ cells after PWM stimulation | > 1,5 | < 1,5 |
| AI2 of CD 19+ cells after PHA stimulation | > 4,5 | < 4,5 |
| AI2 of CD19+ cells after PWM stimulation | > 4,5 | < 4,5 |
| AI1 of CD19+ cells after PWM stimulation | > 1,0 | < 1,0 |
| Percentage of CD28+\|CD8+ | >30% | < 30% |
| Ratio of CD28+ to CD28- on CD8+ T-cells | > 0,5 | < 0,5 |
| Percentage of CD45RA+\|CD4+ | > 50% | < 50% |
| Percentage of CD45RO+\|CD3+ | < 55% | > 55% |
| Ratio of CD45RA+ to CD45RO+ on CD4+ T-cells | > 0,6 | < 0,6 |
| Ratio of CD45RA- to CD45RO- on CD4+ T-cells | < 1,6 | > 1,6 |

Mitogens that are preferred for the methods of the present invention are phytohaemagglutinine (PHA) or pokeweed mitogen (PWM).

In a preferred embodiment of the second diagnostic method of the present invention, said different chronic progressive Non-Alzheimer's Disease is Parkinsons's Disease (PD), dementia with Lewy bodies, Fronto-Temporal Dementia and Vascular Dementia.

In a further preferred embodiment of the diagnostic method of the present invention additionally the expression of CD28 is determined, further enhancing the reliability of the method.

The blood cells, for purposes of illustration and not limitation, may comprise monocytes, macrophages, lymphocytes, or combinations thereof. In a particular embodiment, the blood cells may comprise T cells, B cells, or a combination thereof. In a more preferred embodiment, the blood cells comprise CD3-, CD4-, CD8-, CD14- and CD19-positive cells.

In an even more preferred embodiment of the method of the present invention
(a) the expression of CD69 on CD4-, CD8-, CD14- and/or CD18-positive cells is determined;
(b) the expression of CD45RA respective CD45RO on CD3-and/or CD4-positive cells; and, optionally,
(c) the expression of CD28 on CD8-positive cells is determined.

Any method allowing the determination of the levels of CD69, CD28, CD45RO and CD45RA, respectively, in a sample can be used in the method of the present invention, e.g. immunological assays, size separation, e.g. by gel filtration, amino acid analyses etc. with immunological assays being preferred. For example, expression of said surface markers can be determined by cytometric analysis, Western blot, RT-PCR, or a combination thereof.

Preferably, the determination of the level of the surface markers is carried by an immunological assay using specific antibodies. Suitable antibodies are commercially available, e.g. the following antibodies (obtainable from BD Biosciences, San Jose,CA, USA):
**A** - IgG1-FITC/IgG1-PE/CD45- PerCP-Cy5.5/IgG1-APC;
**B** - CD8-FITC/CD69-PE/CD3-PerCP-Cy5.5/CD4-APC;
**C** - CD14-FITC/CD69-PE/CD45-PerCP-Cy5.5/CD19-APC;
**D** - CD8-FITC/CD28-PE/CD45-PerCP-Cy5.5/CD4-APC; and
**E** - CD45RA-FITC/CD45RO-PE/CD3-PerCP-Cy5.5/CD4-APC.

The term "antibody" as used herein, preferably, relates to antibodies which consist essentially of pooled monoclonal antibodies with different epitopic specificities, as well as distinct monoclonal antibody preparations. Monoclonal antibodies are made from an antigen containing fragments of the hAβ1-42 peptide and hAβ1-40, respectively by methods well known to those skilled in the art. As used herein, the term "antibody" (Ab) or "monoclonal antibody" (Mab) is meant to include intact molecules as well as antibody fragments (such as, for example, Fab and F(ab')2 fragments) which are capable of specifically binding to protein. Fab and F(ab')2 fragments lack the Fc fragment of intact antibody. Moreover, these include chimeric and single chain antibodies.

The probes, e.g. an antibody, can be detectably labeled, for example, with a radioisotope, a bioluminescent compound, a chemiluminescent compound, a fluorescent compound, a metal chelate, or an enzyme (e.g. horse-radish peroxidase, alkaline phosphatase, β-galactosidase, malate dehydrogenase, glucose oxidase, urease, catalase etc.) which, in turn, when later exposed to a substrate will react to the substrate in such a manner as to produce a chemical moiety which can be detected. The probes can also be immobilized on an insoluble carrier, e.g. glass, polystyrene, polypropylene, polyethylene, dextran, nylon, natural and modified celluloses, polyacrylamides, agarose and magnetic beads.

Examples of immunoassays suitable for the methods of the present invention are competitive or sandwich assays, such as the enzyme linked immunosorbent assay (ELISA), the radioimmunoassay (RIA) or Western Blots. Suitable antibody assay labels are known in the art and include enzyme labels, such as, glucose oxidase, and radioisotopes, such as iodine (¹²⁵I, ¹²¹I), carbon (¹⁴C), sulfur (³⁵S), tritium (³H), indium (¹¹²In), and technetium (⁹⁹mTc), and fluorescent labels, such as fluorescein and rhodamine.

In a further preferred embodiment, the methods of the present invention are carried out in an ELISA format with a sandwich-ELISA format being even more preferred. Suitable ELISA and sandwich-ELISA formats are well known to the person skilled in the art.

The method of the present invention can be modified, e.g., by additionally determining the expression of further biomarkers which might, in certain cases, further improve the diagnostic value of the method of the invention.

In a further preferred embodiment of the diagnostic method of the present invention the relating step comprises
(a) comparing the expression of CD69 determined before mitogenic stimulation with the expression after mitogenic stimulation;
(b) determining the ratio of CD45RO/CD45RA; and, optionally,
(c) the expression of CD28,
wherein a substantial impairment of mitogenic response of CD4-, CD8-, CD14-, and/or CD19-positive cells determined by CD69 expression, an increased ratio of CD45RO/CD45RA and, optionally, an increased expression of CD28 compared to that found in cognitively normal patients is indicative of the risk of developing AD, a diagnosis of AD, or a measure of the progression of AD.

In an alternative preferred embodiment of the diagnsotic method of the present invention the relating step comprises
(a) comparing the expression of CD69 determined before mitogenic stimulation with the expression after mitogenic stimulation;
(b) determining the ratio of CD45RO/CD45RA; and, optionally,
(c) the expression of CD28,
wherein a substantial impairment of mitogenic response of CD4-, CD8-, CD14-, and/or CD19-positive cells determined by CD69 expression, an increased ratio of CD45RO/CD45RA and, optionally, an increased expression of CD28 compared to that found in cognitively normal patients is indicative of a Non-AD progressive neurodegenerative disease with dementia, except for Parkinson's Disease (see Tables 1 and 2).

For use in the diagnostic research discussed above, kits are also provided by the present invention. Such kits are useful for the determining the amounts of CD69, CD45RO, CD45RA and, optionally, CD28, in a sample, and comprise specific antibodies for detection. The antibodies can be detectably labeled as described above. In a preferred embodiment, said kit contains an anti-CD69 antibody, an anti-CD45RO-antibody, an anti-CD45RA-antibody and, optionally, an anti-CD28 antibody. Preferably, said antibodies are monoclonal antibodies. Preferably said kit allows diagnosis, e.g., by ELISA and contains the antibodies bound to a solid support, for example, a polystyrene microtiter dish or nitrocellulose paper, using techniques known in the art. Alternatively, said kits are based on a RIA and contain said antibodies marked with a radioactive isotope. In a preferred embodiment of the kit of the invention the antibodies are labeled with enzymes, fluorescent compounds, luminescent compounds, ferromagnetic probes or radioactive compounds.

The following Examples illustrate the invention.

### Example 1

### Materials and Methods

### (A) Participants

92 subjects were recruited for this study. Four subjects were excluded from the study because they did not meet the eligibility criteria. 88 subjects were included in the present study, of which 32 had a clinical diagnosis of probable Alzheimer's disease (AD), 26 were demented and diagnosed with Parkinson's disease (PD) and 30 were classified as cognitively intact subjects (CI). Clinical diagnosis of AD was made according to NINCDS-ADRDA (21) or DSM-IV criteria (Diagnostic and Statistical Manual of Mental Disorders, American Psychiatric Association).

The present study was approved by the IRC institutional review board. Written informed consent was obtained from all participants of the study or their legal representatives.

### (B) Assay methods

Blood samples were taken at four of Pivotal Research Centers' locations by venous puncture into 10ml CPT tubes (heparin/Nacitrate, BD Biosciences, San Jose, CA, USA). All blood cell preparation, staining and analysis was performed at BD Biosciences Pharmingen's Custom Technology Laboratory (La Jolla, CA). Samples were shipped overnight at room temperature and sample preparation was completed within 24 hours after collection.

Peripheral blood mononuclear cells (PBMC) were isolated as described in the product guidelines for CPT tubes and suspended in complete media (BD Pharmingen, San Diego, CA, USA) at 2,5 x 10⁶ cells/ml. Isolated cells were stimulated with Phytohaemagglutinine (PHA-L, 12 *µ*g/ml, Sigma Aldrich St. Louis, MO, USA) and Pokeweed mitogen (PWM, 4 *µ*g/ml, Sigma Aldrich). The non-stimulated reference sample contained an equal volume of complete media. Samples were incubated at 37°C with 7% CO₂ for 4 hours, subsequently mixed with 5 ml of FACS-Lysing solution (BD Biosciences) each, immediately frozen and stored at -70°C. Staining of cells was performed in batch after thawing of samples, with the following antibody-cocktails (custom reagents, BD Biosciences Pharmingen):
**A** - IgG1-FITC/IgG1-PE/CD45- PerCP-Cy5.5/IgG1-APC,
**B** - CD8-FITC/CD69-PE/CD3-PerCP-Cy5.5/CD4-APC,
**C** - CD14-FITC/CD69-PE/CD45-PerCP-Cy5.5/CD19-APC,
**D** - CD8-FITC/CD28-PE/CD45-PerCP-Cy5.5/CD4-APC,
**E** - CD45RA-FITC/CD45RO-PE/CD3-PerCP-Cy5.5/CD4-APC.

The reference sample (non-stimulated) was prepared with each antibody cocktail whereas the stimulated samples were probed with antibody-cocktails B, C and D only. The samples were stored at 2°C to 8°C for a maximum time of 8 hours prior the flow cytometer analysis. Samples were analyzed using a calibrated FacsCalibur Flow cytometer and CellQuest Pro 5.2 software for data acquisition. A total of 1 x 10⁴ gated events were collected for each data file. Blinded raw data analysis was performed by using CellQuest Pro 5.2 and validated.

### (c) Data analysis and statistical evaluation

Raw data analysis was based on the data obtained by flow cytometry. 40 potential markers were specified, involving the determination of two different Activation Indices (AI) that derived from substantially different calculation models. AI1 is defined by the ratio of the percentages of CD69-positive cells after and before stimulation after the determination of a cut-off point between CD69-positive and CD69-negative cells on the basis of a control sample (isotype control). AI2 is defined by the ratio of the mean CD69 expression of the analyzed cell population after and before the stimulation.

Statistical analysis included an evaluation of selected demographic and health factors that could affect the investigated markers of each subject, creation of a normalized data set to account for factors found to be statistically significant, and an analysis of both normalized and raw data collected during blinded flow cytometry. The analysis included data from all 88 subjects who meet the initial eligibility criteria.

First, the hypothesis that each subject's demographic and health characteristics could affect the investigated markers was tested by conducting a multi-iterative linear regression using each marker as a dependent variable and all factors that could affect the corresponding marker as the independent variables. Factors that had a p-value of less than 0.05 were considered significantly different. The coefficients of the significant factors were then used to create a normalized data set that removed mitogenic response due to an ascribable factor.

For both raw and normalized data, cumulative frequency distribution diagrams were constructed and potential cut-points were identified at the maximum difference between AD-probable and Parkinson's disease subjects or all subjects of the non-AD group. When the difference was greater than 15%, discrete variables were created that indicated whether the potential cut-point was exceeded. A student t-Test was then employed to determine if raw, normalized or discrete makers were significantly different between AD subjects and PD subjects or cognitively intact controls or all subjects in the mixed non-AD group, respectively. Markers with a p-value less than 0.05 were considered significantly different. A multi-iterative regression analysis was preformed using the clinical diagnosis as the dependent variable. All markers were used as continuous independent variables and markers with an identified cut-point were used as discrete independent variables. This regression was continued until the adjusted R² was maximized and all remaining variables had p-value of less than 0.05.

### (D) Data Modeling

Several scoring models were generated, comprising either all data from flow cytometry or partial data sets including data resulting from PHA- or PWM-stimulation only. The total data scoring model was based on results obtained by flow cytometry along with demographic and medical history information. Scoring models for the total and the partial data sets were generated using the raw data or the normalized data, respectively. The variables in all models had p-values <0.05.

### Example 2

### Results

### (A) Characterization of the Subjects

88 subjects were included in the study. Subjects of AD, PD and CI group were well matched with regard to age, gender, BMI and health status distributions.

### (B) Outcome measures

40 potential marker were determined by flow cytometry. Demographic factors that were found to affect these markers included the subject's age and gender. Health factors that were found to affect investigated markers included alcohol consumption, arthritis, body mass index, depression and seasonal allergies.

### (C) Statistically Significant Markers

Markers that were found significantly different in AD patients compared to either demented PD subjects or non-AD subjects (p<0.05), respectively, are shown in Tables 1 to 6. The mitogenic response of CD4+, CD14+ and CD19+ cells, determined by CD69 expression analysis, was significantly impaired in AD subjects and tended to have the lowest p-values.

**Table 3**

| **Significant markers differentiating Alzheimer's disease from Parkinson's disease** | | |
|---|---|---|
| **Marker** | **Raw data p-value** | **Normalized data p-value** |
| AI1 of CD8+ T-cells after PWM stimulation * | 0.005 | 0.013 |
| AI1 of CD14+ cells after PWM stimulation * | - | 0.037 |
| AI2 of CD14+ cells after PHA stimulation | - | 0.002 |
| AI2 of CD14+ cells after PWM stimulation* | 0.016 | 0.002 |
| A12 of CD19+ cells after PHA stimulation | 0.025 | 0.035 |
| AI2 of CD19+ cells after PWM stimulation | 0.009 | 0.009 |
| AI2 of CD19+ cells after PWM stimulation * | 0.016 | 0.005 |
| AI1 of CD19+ cells after PWM stimulation* | 0.005 | 0.002 |
| Percentage of CD28+\|CD8+ * | 0.041 | 0.041 |
| Ratio of CD28+ to CD28- on CD8+ T-cells * | 0.041 | N/A |
| Percentage of CD45PA+\|CD4+ * | - | 0.021 |
| Percentage of CD45RO+\|CD3+* | 0.036 | 0.043 |
| Ratio of CD45RA+ to CD45RO+ on CD4+ T-cells * | 0.031 | 0.031 |
| Ratio of CD45RA- to CD45RO- on CD4+ T-cells * | 0.040 | N/A |
| * Denotes discrete variable based on value exceeding cut-point. | | |

**Table 4**

| **Significant markers differentiating Alzheimer's disease from non-Alzheimer's disease.** | | |
|---|---|---|
| **Marker** | **Raw data p-value** | **Normalized data p-value** |
| Percentage of CD45RO+\|CD3+ * | - | 0.013 |
| AI2 of CD4+ T-cells after PWM stimulation * | - | 0.041 |
| AI1 of CD4+ T-cells after PHA stimulation * | 0.023 | 0.009 |
| AI1 of CD8+ T-cells after PWM stimulation * | 0.018 | 0.018 |
| AI2 of CD8+ T-cells after PHA stimulation * | 0.02 | 0.009 |
| AI1 of CD14+ cells after PWM stimulation | 0.030 | 0.028 |
| AI1 of CD14+ cells after PHA stimulation * | 0.013 | 0.037 |
| AI2 of CD14+ cells after PHA stimulation * | 0.018 | 0.009 |
| AI2 of CD14+ cells | 0.004 | 0.004 |
| AI2 of CD14+ cells after PWM stimulation * | 0.005 | 0.003 |
| AI1 of CD19+ cells after PWM stimulation | 0.002 | 0.006 |
| AI2 of CD19+ cells after PWM stimulation | 0.007 | 0.007 |
| AI2 of CD19+ cells after PWM stimulation * | - | 0.002 |
| AI1 of CD19+ cells after PWM stimulation * | 0.009 | 0.004 |
| AI2 of CD19+ cells after PHA stimulation | 0.008 | 0.004 |
| AI2 of CD19+ cells after PHA stimulation * | - | 0.032 |
| Percentage of CD28+\|CD45+ * | 0.041 | 0.041 |
| * Denotes discrete variable based on value exceeding cut-point. | | |

### (D) Model Performance

Applying the total data set models based on either raw or normalized data it could be differentiated between AD subjects and PD subjects with a co-positivity of 91% and a co-negativity of 92%, and between AD subjects and non-AD subjects with a co-positivity of 88% and a co-negativity of 82%. Single stimulant scoring models based on either PHA- or PWM-stimulation data achieved comparable results. Data are summarized in Table 5. Data from all models were used to construct Receiver Operator Characteristic (ROC) curves that are shown in Figure 1.

**Table 5**

| **Performance comparison of determined scoring models** | | | | | | | | |
|---|---|---|---|---|---|---|---|---|
| | | | **Cut-point** | | | | | |
| **Model differentiates** | **Scoring model based on** | | **0.4** | **0.45** | **0.5** | **0.55** | **0.6** | **0.65** |
| AD vs. PD | Total raw data set | Co-Positive | 94% | 91% | 91% | 91% | 84% | 81% |
| | | Co-Negative | 85% | 88% | 88% | 92% | 92% | 96% |
| | | | | | | | | |
| AD vs. PD | Total normalized | Co-Positive | 97% | 94% | 94% | 91% | 88% | 88% |
| | data set | Co-Negative | 73% | 81% | 88% | 92% | 96% | 96% |
| AD vs. PD | PHA restricted raw | Co-Positive | 97% | 94% | 94% | 91% | 91% | 81% |
| | data set | Co-Negative | 73% | 77% | 85% | 92% | 96% | 96% |
| | | | | | | | | |
| AD vs. PD | PHA restricted | Co-Positive | 97% | 97% | 97% | 97% | 94% | 88% |
| | normalized data set | Co-Negative | 73% | 73% | 85% | 85% | 92% | 96% |
| AD vs. PD | PWM restricted raw | Co-Positive | 100% | 100% | 97% | 94% | 94% | 94% |
| | data set | Co-Negative | 81% | 81% | 88% | 88% | 92% | 92% |
| | | | | | | | | |
| AD vs. PD | PWM restricted | Co-Positive | 100% | 94% | 94% | 91% | 91% | 88% |
| | normalized data set | Co-Negative | 81% | 100% | 100% | 100% | 100% | 100% |
| AD vs. non-AD | Total raw | Co-Positive | 91% | 81% | 69% | 69% | 63% | 59% |
| | data set | Co-Negative | 79% | 88% | 91% | 93% | 95% | 95% |
| | | | | | | | | |
| AD vs. non-AD | Total normalized | Co-Positive | 91% | 88% | 78% | 78% | 72% | 66% |
| | data set | Co-Negative | 79% | 82% | 88% | 91% | 93% | 93% |

### (E) Scoring Model Performance Using Individual Markers

To further evaluate the synergistic effect of the simultaneous detection of various AD biomarkers the performance of individual marker scoring models compared to the total data set scoring model was tested, that used data from flow cytometry, along with demographic and medical history information. Each of the three individual markers retained statistical significance and some level of descriptive power when evaluated separately, whereas mitogenic response related markers providing the highest level of predictive power and CD28-related providing the least, as shown in Table 6. Sensitivity was slightly increased when all flow cytometry based data were combined. However, highest accuracy level was achieved with the scoring model that was based on data obtained by flow cytometry in combination with demographic and medical history data.

**Table 6**

| **Performance of specific marker scoring models compared to the Total data set scoring model, that comprise all data obtained by flow cytometry along with demographic and medical history information.** | | | |
|---|---|---|---|
| **Scoring Model based on** | **Cut point** | **Sensitivity** | **Specificity** |
| Total data set scoring model | 0.55 | 91% | 92% |
| All flow cytometer markers | 0.55 | 88% | 77% |
| Mitogenic response markers | 0.55 | 84% | 77% |
| CD45RA\|CD45RO related markers | 0.5 | 88% | 58% |
| CD28 related markers | 0.5 | 88% | 53% |

### (F) Discussion

The evident advantages of blood specimen collection and mounting evidence of cell cycle dysregulation in AD were the impetus to test whether a biomarker profile of peripheral lymphocytes stimulated with mitogenic compounds will correlate with the clinical diagnosis of AD with an acceptable diagnostic accuracy.

The use of peripheral lymphocytes as diagnostic tool for AD is based on the hypothesis that (i) cell cycle dysregulation represents a mechanism which is critically involved in AD pathology (43-45) and (ii) is not restricted to the brain but rather represents a systemic aspect of the disease also relevant to peripheral cells such as blood cells and fibroblasts (14;15;19;20;29-32). The test system further considers findings on disease-related alterations on lymphocytes that can be determined by flow cytometry and might potentially be relevant explaining potential deficiencies of the immune response of patients (14;31-35).

In the present study, a total of 88 subjects was included, 32 with clinically diagnosed probable AD, 26 demented subjects, diagnosed with Parkinson's disease (PD) and 30 non-demented subjects. PD subjects group was included, as PD is a further form of a progressive neurodegenerative disease that in most cases is associated with dementia. Basis of the present test was the analysis of the expression of CD69 on CD45-, CD3-, CD4-, CD8-, CD19- and CD14-positive cells before and after mitogenic stimulation; the expression of CD28 on CD45-, CD4- and CD8-positive cells and the expression of CD45RA respective CD45RO on CD3- and CD4-positive cells. Using the acquired data we determined 40 markers. Markers that were found to be influenced by demographic or health status factors were normalized, albeit with a population to small to define strong normalization coefficients. However, normalization did not affect test performance.

Mitogenic stimulation was performed using phytohaemagglutinine (PHA) or pokeweed mitogen (PWM) to test the performance of the applied mitogens. The total data set scoring model included both Activation indices (AI) resulting from PHA-stimulation and PWM-stimulation. To further improve practicability and cost efficiency of a potential biomarker test we analyzed scoring models based on only PHA-stimulation or PWM-stimulation respectively. These single stimulant scoring models resulted in test accuracy levels comparable to the total data set scoring model, producing areas under ROC curves of 0.966 and 0.976, respectively (Figure 1).

As with almost every AD biomarker study, the present test is limited by the fact that test results were correlated to clinical diagnosis of AD, which introduces errors in data analysis due to clinical uncertainty, even when subjects are recruited and enrolled by specialists in neurodegenerative diseases. Longitudinal studies are needed that compares test results to the neuropathological confirmation of clinically diagnosed AD cases to conclusively determine sensitivity and specificity of the test. An explicit goal of this study was to evaluate whether the entire test procedure works reliably not only under experimental laboratory conditions or at the level of a clinical center but also under conditions of decentralized units at the primary care level which requires storage and transport of specimens. In the present study blood samples were obtained at five different Pivotal Research locations and shipped over night to a central laboratory (BD Biosciences Pharmingen, San Diego) where they were processed and analyzed meeting requirements necessary to apply the test in a large scale on the primary care level.

### Conclusions

At present, no single biomarker or panel of biomarkers has been shown to be successful as an adjunct to or potential replacement for the clinical diagnosis of AD. A biomarker test that is based on blood samples has distinct advantages. Taking a blood sample is simple, non-invasive procedure (as defined by the FDA's IDE requirements) that may be performed at the primary care level. Primarily based on findings suggesting a critical role for cell cycle dysfunction in AD pathogenesis, in the present study a biomarker profile could be established which shows a co-positivity of 91% and a co-negativity of 92% relative to the clinical diagnosis of AD. This high level of agreement with the clinical diagnosis indicates the test's potential as a diagnostic tool for AD.

### List of references

(1) Cummings JL, Donohue JA, Brooks RL. The relationship between donepezil and behavioral disturbances in patients with Alzheimer's disease. Am J Geriatr Psychiatry 2000; 8(2):134-140.
(2) DeKosky S. Early intervention is key to successful management of Alzheimer disease. Alzheimer Dis Assoc Disord 2003; 17 Suppl 4:S99-104.:S99-104.
(3) Mayeux R, Saunders AM, Shea S, Mirra S, Evans D, Roses AD et al. Utility of the apolipoprotein E genotype in the diagnosis of Alzheimer's disease. Alzheimer's Disease Centers Consortium on Apolipoprotein E and Alzheimer's Disease. N Engl J Med 1998; 338(8):506-511.
(4) Varma AR, Snowden JS, Lloyd JJ, Talbot PR, Mann DM, Neary D. Evaluation of the NINCDS-ADRDA criteria in the differentiation of Alzheimer's disease and frontotemporal dementia. J Neurol Neurosurg Psychiatry 1999; 66(2):184-188.
(5) Blennow K, Vanmechelen E. Combination of the different biological markers for increasing specificity of in vivo Alzheimer's testing. J Neural Transm Suppl 1998; 53:223-35.:223-235.
(6) Thal LJ, Kantarci K, Reiman EM, Klunk WE, Weiner MW, Zetterberg H et al. The Role of Biomarkers in Clinical Trials for Alzheimer Disease. Alzheimer Dis Assoc Disord 2006; 20 (1) :6-15.
(7) Frank RA, Galasko D, Hampel H, Hardy J, de Leon MJ, Mehta PD et al. Biological markers for therapeutic trials in Alzheimer's disease. Proceedings of the biological markers working group; NIA initiative on neuroimaging in Alzheimer's disease. Neurobiol Aging 2003; 24(4):521-536.
(8) Consensus report of the Working Group on: "Molecular and Biochemical Markers of Alzheimer's Disease". The Ronald and Nancy Reagan Research Institute of the Alzheimer's Association and the National Institute on Aging Working Group. Neurobiol Aging 1998; 19(2):109-116.
(9) Arendt T, Holzer M, Gartner U, Bruckner MK. Aberrancies in signal transduction and cell cycle related events in Alzheimer's disease. J Neural Transm Suppl 1998; 54:147-58.:147-158.
(10) Busser J, Geldmacher DS, Herrup K. Ectopic cell cycle proteins predict the sites of neuronal cell death in Alzheimer's disease brain. J Neurosci 1998; 18(8):2801-2807.
(11) Herrup K, Neve R, Ackerman SL, Copani A. Divide and die: cell cycle events as triggers of nerve cell death. J Neurosci 2004; 24(42):9232-9239.
(12) Nagy Z, Esiri MM, Cato AM, Smith AD. Cell cycle markers in the hippocampus in Alzheimer's disease. Acta Neuropathol (Berl) 1997; 94(1):6-15.
(13) Bongioanni P, Boccardi B, Borgna M, Rossi B. T-lymphocyte interleukin 6 receptor binding in patients with dementia of Alzheimer type. Arch Neurol 1998; 55(10):1305-1308.
(14) Ikeda T, Yamamoto K, Takahashi K, Yamada M. Immune system-associated antigens on the surface of peripheral blood lymphocytes in patients with Alzheimer's disease. Acta Psychiatr Scand 1991; 83(6):444-448.
(15) Inestrosa NC, Alarcon R, Arriagada J, Donoso A, Alvarez J, Campos EO. Blood markers in Alzheimer disease: subnormal acetylcholinesterase and butyrylcholinesterase in lymphocytes and erythrocytes. J Neurol Sci 1994; 122(1):1-5.
(16) Mecocci P, Polidori MC, Ingegni T, Cherubini A, Chionne F, Cecchetti R et al. Oxidative damage to DNA in lymphocytes from AD patients. Neurology 1998; 51(4):1014-1017.
(17) Mecocci P, Polidori MC, Cherubini A, Ingegni T, Mattioli P, Catani M et al. Lymphocyte oxidative DNA damage and plasma antioxidants in Alzheimer disease. Arch Neurol 2002; 59(5):794-798.
(18) Morocz M, Kalman J, Juhasz A, Sinko I, McGlynn AP, Downes CS et al. Elevated levels of oxidative DNA damage in lymphocytes from patients with Alzheimer's disease. Neurobiol Aging 2002; 23(1):47-53.
(19) Shalit F, Sredni B, Brodie C, Kott E, Huberman M. T lymphocyte subpopulations and activation markers correlate with severity of Alzheimer's disease. Clin Immunol Immunopathol 1995; 75(3):246-250.
(20) Zhang J, Kong Q, Zhang Z, Ge P, Ba D, He W. Telomere dysfunction of lymphocytes in patients with Alzheimer disease. Cogn Behav Neurol 2003; 16(3):170-176.
(21) Tierney MC, Fisher RH, Lewis AJ, Zorzitto ML, Snow WG, Reid DW et al. The NINCDS-ADRDA Work Group criteria for the clinical diagnosis of probable Alzheimer's disease: a clinicopathologic study of 57 cases. Neurology 1988; 38 (3) :359-364.
(22) McKhann G, Drachman D, Folstein M, Katzman R, Price D, Stadlan EM. Clinical diagnosis of Alzheimer's disease: report of the NINCDS-ADRDA Work Group under the auspices of Department of Health and Human Services Task Force on Alzheimer's Disease. Neurology 1984; 34(7):939-944.
(23) Maccioni RB, Lavados M, Maccioni CB, Mendoza-Naranjo A. Biological markers of Alzheimer's disease and mild cognitive impairment. Curr Alzheimer Res 2004; 1(4):307-314.
(24) Andreasen N, Blennow K. CSF biomarkers for mild cognitive impairment and early Alzheimer's disease. Clin Neurol Neurosurg 2005; 107(3):165-173.
(25) Mendez MF, Mastri AR, Sung JH, Frey WH. Clinically diagnosed Alzheimer disease: neuropathologic findings in 650 cases. Alzheimer Dis Assoc Disord 1992; 6(1):35-43.
(26) Arendt T. Synaptic plasticity and cell cycle activation in neurons are alternative effector pathways: the 'Dr. Jekyll and Mr. Hyde concept' of Alzheimer's disease or the yin and yang of neuroplasticity. Prog Neurobiol 2003; 71 (2-3) :83-248.
(27) Hulstaert F, Blennow K, Ivanoiu A, Schoonderwaldt HC, Riemenschneider M, De Deyn PP et al. Improved discrimination of AD patients using beta-amyloid(1-42) and tau levels in CSF. Neurology 1999; 52(8):1555-1562.
(28) Irizarry MC. Biomarkers of Alzheimer disease in plasma. NeuroRx 2004; 1(2):226-234.
(29) Emiliani C, Urbanelli L, Racanicchi L, Orlacchio A, Pelicci G, Sorbi S et al. Up-regulation of glycohydrolases in Alzheimer's Disease fibroblasts correlates with Ras activation. J Biol Chem 2003; 278(40):38453-38460.
(30) Hirashima N, Etcheberrigaray R, Bergamaschi S, Racchi M, Battaini F, Binetti G et al. Calcium responses in human fibroblasts: a diagnostic molecular profile for Alzheimer's disease. Neurobiol Aging 1996; 17(4):549-555.
(31) Lombardi VR, Garcia M, Rey L, Cacabelos R. Characterization of cytokine production, screening of lymphocyte subset patterns and in vitro apoptosis in healthy and Alzheimer's Disease (AD) individuals. J Neuroimmunol 1999; 97(1-2):163-171.
(32) Stieler JT, Lederer C, Bruckner MK, Wolf H, Holzer M, Gertz HJ et al. Impairment of mitogenic activation of peripheral blood lymphocytes in Alzheimer's disease. Neuroreport 2001; 12(18):3969-3972.
(33) Tan J, Town T, Abdullah L, Wu Y, Placzek A, Small B et al. CD45 isoform alteration in CD4+ T cells as a potential diagnostic marker of Alzheimer's disease. J Neuroimmunol 2002; 132(1-2):164-172.
(34) Kusdra L, Rempel H, Yaffe K, Pulliam L. Elevation of CD69+ monocyte/macrophages in patients with Alzheimer's disease. Immunobiology 2000; 202(1):26-33.
(35) Pirttila T, Mattinen S, Frey H. The decrease of CD8-positive lymphocytes in Alzheimer's disease. J Neurol Sci 1992; 107(2):160-165.

## Claims

1. A method of diagnosing Alzheimer's Disease (AD), an early stage or predisposition for said disease in a blood sample obtained from a patient comprising:
(a) quantitatively determining (i) expression of blood cell surface marker CD69 prior to and after stimulation of the cells of the blood sample by a mitogen and (ii) the ratio of CD45RO/CD45RA expression;
(b) comparing the expression values of said surface markers of the blood cells with reference expression values of the same surface markers; and
(c) relating the determined expression of CD69 and the ratio of CD45RO/CD45RA to an assessment of the risk of developing the disease, a diagnosis of the disease, or a measure of the progression of the disease.

2. A diagnostic method for distinguishing between AD and a different chronic progressive Non-Alzheimer's Dementia comprising:
(a) quantitatively determining (i) expression of blood cell surface marker CD69 prior to and after stimulation of the cells of the blood sample between by a mitogen and (ii) the ratio of CD45RO/CD45RA;
(b) comparing the expression values of said surface markers of the blood cells with reference expression values of the same surface markers; and
(c) relating the determined expression of CD69 and the ratio of CD45RO/CD45RA for distinguishing AD and a different chronic progressive Non-Alzheimer's Dementia.

3. The method of claim 1 or 2, wherein said mitogen is phytohaemagglutinine (PHA) or pokeweed mitogen (PWM).

4. The method of claim 2, wherein said different chronic progressive Non-Alzheimer's Dementia is Parkinson's Disease (PD).

5. The method of any one of claims 1 to 4, further comprising the determination of the expression of the cell surface marker CD28.

6. The method of any one of claims 1 to 5, wherein said blood cells comprise monocytes macrophages, lymphocytes, or combinations thereof.

7. The method of any one of claims 1 to 6, wherein said blood cells comprise T cells, B cells, or a combination thereof.

8. The method of any one of claims 1 to 7, wherein said blood cells comprise CD3-, CD4-, CD8-, CD14- and CD19-positive cells.

9. The method of claim 8, comprising determining:
(a) the expression of CD69 on CD4-, CD8-, CD14- and/or CD19-positive cells;
(b) the expression of CD45RA respective CD45RO on CD3- and/or CD4-positive cells; and, optionally,
(c) the expression of CD28 on CD8-positive cells.

10. The method of any one of claims 1 to 9, wherein expression of said surface markers is determined by cytometric analysis, Western blot, RT-PCR, or a combination thereof.

11. The method of any one of claims 1 or 3 to 10, wherein the relating step comprises
(a) comparing the expression of CD69 determined before mitogenic stimulation with the expression after mitogenic stimulation;
(b) determining the ratio of CD45RO/CD45RA; and, optionally,
(c) the expression of CD28,
wherein a substantial impairment of mitogenic response of CD4-CD8-, CD14-, and/or CD19-positive cells determined by CD69 expression, an increased ratio of CD45RO/CD45RA and, optionally, an increased expression of CD28 compared to that found in cognitively normal patients is indicative of the risk of developing AD, a diagnosis of AD, or a measure of the progression of AD.

12. The method of any one of claims 2 to 10, wherein the relating step comprises
(a) comparing the expression of CD69 determined before mitogenic stimulation with the expression after mitogenic stimulation;
(b) determining the ratio of CD45RO/CD45RA; and, optionally,
(c) the expression of CD28,
wherein a substantial impairment of mitogenic response of CD4-, CD8-, CD14-, and/or CD19-positive cells determined by CD69 expression, an increased ratio of CD45RO/CD45RA and, optionally, an increased expression of CD28 compared to that found in cognitively normal patients is indicative of a Non-AD progressive neurodegenerative disease with dementia, except for Parkinson's Disease.

13. Kit suitable for carrying out a method according to any one of claims 1 to 12 comprising an anti-CD69 antibody, an anti-CD45RO-antibody and an anti-CD45RA-antibody.

14. The kit of claim 13, further comprising an anti-CD28 antibody.

15. The kit of claim 13 or 14, further comprising a mitogen.
